(19) Europäisches Patentamt / European Patent Office / Office européen des brevets

(11) **EP 1 425 301 B1**

(12) **FASCICULE DE BREVET EUROPEEN**

(45) Date de publication et mention de la délivrance du brevet:
**14.11.2007 Bulletin 2007/46**

(21) Numéro de dépôt: **02785499.1**

(22) Date de dépôt: **13.09.2002**

(51) Int Cl.:
*C07K 14/44* (2006.01)    *A61K 39/008* (2006.01)
*G01N 33/68* (2006.01)

(86) Numéro de dépôt international:
**PCT/FR2002/003134**

(87) Numéro de publication internationale:
**WO 2003/025012 (27.03.2003 Gazette 2003/13)**

(54) **MELANGE VACCINAL THERAPEUTIQUE DESTINE A LA PREVENTION ET AU TRAITEMENT D'AFFECTIONS CHEZ LES MAMMIFERES**

THERAPEUTISCHE IMPFZUSAMMENSETZUNG ZUR VERHÜTUNG UND BEHANDLUNG VON ERKRANKUNGEN BEI SÄUGETIEREN

THERAPEUTIC VACCINE MIXTURE FOR PREVENTING AND TREATING DISORDERS IN MAMMALS

(84) Etats contractants désignés:
**AT BE BG CH CY CZ DE DK EE ES FI FR GB GR IE IT LI LU MC NL PT SE SK TR**

(30) Priorité: **14.09.2001 FR 0111942**

(43) Date de publication de la demande:
**09.06.2004 Bulletin 2004/24**

(73) Titulaire: **Oridan, Inc.**
**Wilmington, DE 19810 (US)**

(72) Inventeurs:
- **PAPIEROK, Gérard,**
  **Résidence Reine Victoria**
  **83400 Hyères (FR)**
- **VINCENS, Serge**
  **13180 Gignac La Nerthe (FR)**

(74) Mandataire: **Marek, Pierre**
**Cabinet Marek,**
**28 - 32, rue de la Loge**
**13002 Marseille (FR)**

(56) Documents cités:
**WO-A-97/48725**

- **LOHMAN KL ET AL.: "Molecular cloning and characterization of the immunologically protective surface glycoprotein GP46/M-2 of Leishmania amazonensis" PROCEEDINGS OF THE NATIONAL ACADEMY OF SCIENCE USA, vol. 87, novembre 1990 (1990-11), pages 8393-8397, XP002204079**
- **PUENTES F ET AL.: "Leishmania: Fine Mapping of the Leishmanolysin Molecule's Conserved Core Domains Involved in Binding and Internalization" EXPERIMENTAL PARASITOLOGY, vol. 93, 1999, pages 7-22, XP001086537**
- **SOTO M ET AL.: "Antigenicity of the Leishmania infantum histones H2B and H4 during canine viscerocutaneous leishmaniasis" CLINICAL AND EXPERIMENTAL IMMUNOLOGY, vol. 115, février 1999 (1999-02), pages 342-349, XP001086535**
- **CHAMPSI J & MCMAHON-PRATT D: INFECTION AND IMMUNITY, vol. 56, 1988, pages 3272-3279,**

**Description**

Mélange vaccinal thérapeutique destiné à la prévention et au traitement d'affections chez les mammifères et en particulier chez l'homme, les canidés, les félidés et les équidés dont l'immunité protectrice dépend de la stimulation de lymphocytes T de type Th1 et notamment d'un état d'hypersensibilité de type retardée.

Mélange destiné également au diagnostic de l'immunité à médiation cellulaire dépendant de lymphocytes T de type Th1 utilisant diverses méthodes « in vivo » et « in vitro ».

Au cours de la réponse immunitaire, le lymphocyte T constitue une source importante de cytokines. Leur production est induite après stimulation de façon spécifique en présence d'antigènes ou non spécifique en présence de mitogènes (concanaviline A, phytohémagglutinine).

S'il est clairement établi que les lymphocytes T, et parmi eux surtout les lymphocytes CD4+, représentent la source principale de cytokines, les sous-populations T impliquées dans ce phénomène sont diverses et semblent varier en fonction du stimulus.

On peut diviser de manière très schématique les réponses immunitaires en deux grandes catégories qualitativement distinctes, les réponses humorales qui mettent en jeu la production d'anticorps par les lymphocytes B et les réponses cellulaires (réaction d'hypersensibilité retardée, réactions cytotoxiques) pour lesquelles les cellules effectrices sont des lymphocytes T. Il apparaît que dans la majorité des modèles expérimentaux et des situations cliniques étudiées, les cytokines d'origine lymphocytaire sont essentiellement produites par les cellules T auxilaires CD4+ (ou helper) dont le rôle est de moduler ou de réguler l'immunité humorale et cellulaire et qui reconnaissent l'antigène en association avec les molécules de classe II du complexe majeur d'histocompatibilité. Un concept majeur a émergé en 1985 lorsque T. Mosmann et R.Coffman ont proposé que les cellules lymphocytaires T CD4+ exprimant des fonctions auxiliaires étaient en fait hétérogènes. Ainsi, grâce à l'étude de clones lymphocytaires T CD4+ de souris, cultivés à long terme, ces auteurs ont décrit l'existence de deux sous-populations majeures que l'on peut distinguer grâce à leur profil de sécrétion de cytokines à savoir les cellules $Th_1$ (pour T helper de type 1) et les cellules $Th_2$ (pour T helper de type 2).

Prenons comme exemple la leishmaniose, qui est une infection parasitaire endémique voire épidémique de régions tropicales et subtropicales du monde. Les *leishmania,* protozoaires flagellés de la famille des trypanosomatidae et du genre *Leishmania,* sont les agents pathogènes responsables de ces maladies.

Les nombreuses études portant sur les réponses immunitaires au cours des leishmanioses murines expérimentales ont conduit à la démonstration du rôle prépondérant de l'immunité à médiation cellulaire et de l'existence d'une dualité de la réponse immunologique. Il existe fondamentalement deux types de réponses à l'encontre des leishmanies : l'une qualifiée de « sensibilité », l'autre de « résistance ». C'est par le biais des lymphokines qu'elles sécrètent que les différentes sous-populations de lymphocytes T (CD4+) limitent ou exacerbent l'infection. Il a ainsi été démontré que la sous-population de lymphocytes T auxiliaires de type Th1 (producteur d'interféron gamma et d'interleukine 2) était capable d'éliminer les formes amastigotes intracellulaires par le biais de l'activation des macrophages (Reiner S.L et al., Annu Rev Immunol, 1995, 13, 151-177. Review). Inversement la sous population de lymphocytes T auxiliaires de type Th2 (producteur d'interleukine 4) est responsable de l'exacerbation de la maladie.

Chez l'homme, certains faits sont de nature comparable. Chez le chien (hôte naturel « réservoir » réceptif au cycle évolutif de *L.infantum*), la dualité de la réponse immunologique est vraisemblable. Seule une étude menée par Pinelli et al. (Infect. Immun., 62 :229, 1994) sur des animaux expérimentalement et naturellement infectés par *L.infantum,* a permis de montrer que l'asymptomatisme du chien (état clinique fréquemment rencontré) s'accompagnait de l'absence d'une réponse humorale et du développement d'une immunité à médiation cellulaire de type Th1 avec une réaction d'hypersensibilité de type retardée positive et des taux élevés d'interleukine 2 et de TNF-$\alpha$ circulant dans les liquides biologiques.

Un bon candidat vaccinal se doit donc de correspondre à un ou plusieurs antigènes parasitaires fortement immunogène(s) capables soit de bloquer la différenciation des lymphocytes Th2 (Gurunathan S et al., J.Exp Med, 1997 Oct 6, 186, 1137-1147) (mode d'intervention comparable aux traitements de « désensibilisation », couramment pratiqués dans les cas d'allergie), soit de favoriser l'émergence de lymphocytes Th1 assurant la mise en place d'une immunité protectrice.

Envisager de vacciner contre les leishmanies reste encore aujourd'hui problématique. Les tentatives sont nombreuses mais les résultats sont faibles et/ou contradictoires. Nous pouvons citer l'utilisation de parasites vivants, de parasites irradiés et de parasites tués entiers (Moreau Y et coll, 1994, Médecine et Armées, 22, 1, 89-93) qui ont donné des niveaux de protection variable chez les souris et chez l'homme.

Lohman et al. (1990), Proceedings of the National Academy of Science USA 87: 8393-8397, se rapportent au clonage et au séquençage du gène encodant pour la protéine native GP42/M2 de la forme promastigote de Leishmania amazonensis, et relèvent l'intérêt de cette protéine pour des études vaccinales futures, en raison de son implication dans l'immunisation contre les leishmanioses notamment de forme cutanée, chez les modèles expérimentaux murins.

Dans les années 80, des extraits purifiés d'antigènes parasitaires ont été utilisés chez le chien en induisant une exacerbation de la maladie : fraction LIF2 et vaccin anti-idiotypique de l'équipe du Dr Montjour. (CHAUVY, J « Essais d'immunothérapie sur une population canine en zone d'endémie leishmanienne » thèse n°36.1993-OGUNKOLADE B.W et coll. Vet Parasitol.), GP63 ou lipophosphoglucane (MOREAU Y et coll, Médecine et Armées, 1994, 22,1, 89-93) n'ont pas donné de résultat satisfaisant. Actuellement, plusieurs molécules sont à l'essai et sont en attente de résultat final. Citons la protéine de heat shock HSP83 de *Leishmania major* qui stimule la voie Th1 et la protéine DP72 (JAFFE.C et al, J of Immunol, 1990, 144, 699-706). Cependant, aucun des protocoles actuels d'immunisation ne permet d'obtenir un niveau suffisant de protection ou n'est en tout cas reproductible.

A ce jour, aucun travail n'a été effectué avec des peptides de synthèse.

La présente invention consiste en un mélange immunomodulateur utilisant un ou deux peptides avec un adjuvant induisant soit une immunostimulation du système lymphocytaire T de type Th1 de façon reproductible, soit une immunomodulation d'une réponse de type Th2 vers celle de type Th1.

Il est important de souligner deux faits essentiels. Premièrement, les cellules Th1 et Th2 exercent, par le biais de certaines des cytokines qu'elles produisent, un effet de contrerégulation réciproque, ce qui explique pourquoi dans la majorité des cas on observe une alternance de réponses à médiation cellulaire et humorale. Ainsi, les réponses immunitaires qui prévilegient une réaction de lymphocytes T de type Th1, c'est à dire avec des organismes induisant une immunité dépendant des lymphocytes T, sont fréquemment associées à une faible réponse humorale ; c'est le cas par exemple des infections mycobactériennes.

Inversement, une réponse qui privilégie la production d'anticorps sera le plus souvent associée soit à la participation des lymphocytes T de type Th2 soit à un déficit relatif de l'immunité cellulaire spécifique ; c'est le cas de la leishmaniose viscérale. Deuxième donnée fondamentale, les lymphocytes pleinement différenciés en cellules de type Th1 et Th2 ne préexistent pas chez l'hôte naïf non sensibilisé. Au cours d'une réponse immunitaire physiologique suite à laquelle, le plus souvent, les antigènes sont rapidement éliminés, les cellules CD4$^+$ activées, plutôt que de s'exprimer soit par un phénotype de type Th1 soit par un phénotype de type Th2 sécrètent habituellement des cytokines spécifiques de type Th1 et Th2. C'est ce qui explique que, chez l'homme, la démonstration de l'existence d'une dichotomie Th1/Th2 a nécessité l'étude d'un réseau cytokinique à partir de lymphocytes non pas de sujets normaux normalement immunisés mais de patients présentant des maladies chroniques telles des infections parasitaires, des allergies ou des maladies auto-immunes.

La polarisation des réponses immunitaires vers un phénotype Th1 ou Th2 a été associée à de nombreuses situations pathologiques.

Pour les infections à *leishmania, Trypanozoma, Candida,* et autres organismes intracellulaires comme *Mycobacterium* et *Listeria* une réponse Th1 corrèle avec la résistance au pathogène. D'autre part, certaines affections comme les dermites atopiques canines, les allergies et l'asthme conduisent à l'exacerbation d'une réponse de type Th2. Le recours à des immunostimulants permettant le passage d'une réponse Th2 à une réponse Th1, donc le passage d'un état d'hypersensibilité immédiate à un état d'hypersensibilité de type retardée devrait induire une guérison.

Prenons comme exemple l'asthme pour laquelle la communauté médicale s'inquiète devant le nombre croissant d'individus qui en souffre : environ 200 millions de personnes dans le monde et selon l'OMS 180000 en sont mortes en 1997. En France 2000 personnes meurent d'asthme chaque année.

L'asthme est un état inflammatoire chronique des voies respiratoires où interviennent de nombreuses cellules du système immunitaire.

La maladie devient chronique à cause de l'accumulation quasi permanente de médiateurs de l'inflammation, ainsi que du recrutement quasi incessant de polynucléaires éosinophiles destructeurs : ces cellules s'accumulent dans la muqueuse bronchique et libèrent des protéines basiques qui détruisent l'épithélium bronchique. Les dégâts que causent ces protéines basiques entraînent une hyperactivité des bronches : les terminaisons nerveuses qui innervent les bronches sont mises à nu et ne sont plus protégées des agressions extérieures.

Rappelons que les lymphocytes T activés libèrent notamment de l'interleukine 4, qui déclenche la production d'immunoglobulines E. Cette « orientation allergique » encore nommée voie Th2, s'oppose aux réactions anti-infectieuses de la voie Th1 : dans le cadre de la lutte contre une bactérie (le bacille de la tuberculose, par exemple), les lymphocytes T activés libèrent de l'interleukine 2 et de l'interféron gamma. L'interféron gamma active les cellules présentatrices d'antigène qui, à leur tour, produisent de l'interleukine 12. Ainsi, l'interleukine 4 oriente les réactions immunitaires vers les réactions allergiques, tandis que l'interféron gamma semble promouvoir les réactions de type défense.

Or cette orientation du système immunitaire serait imprimée très tôt, chez le tout jeune enfant. Chez un nourrisson exposé à un agent pathogène, le système immunitaire s'oriente vers la voie défensive : ce sont les lymphocytes T producteurs d'interleukine 2 et d'interféron gamma qui sont activés les premiers et qui le resteront. Ces enfants auront moins de risques allergiques.

En revanche, des nourrissons élevés en milieu aseptisé ont des risques d'être confrontés à un environnement riche en allergènes et pauvres en micro-organismes pathogènes.

Si tel est le cas, le système immunitaire encore immature va activer les lymphocytes T qui sécrètent l'interleukine 4, conférant au système immunitaire une orientation allergique, laquelle favorise l'asthme. Cette hypothèse, issue des travaux de Tim Mosman et Robert Coffann, de l'institut de biologie moléculaire et cellulaire DNAX, à Palo Alto, expliquerait l'augmentation de la prévalence de la maladie asthmatique dans les pays industrialisés.

Une réorientation des réactions immunitaires de personnes sensibles en limitant la voie allergique Th2 de la réponse immunitaire vers une activation de la voie Th1 serait une possibilité de traitement de l'asthme.

Nous pouvons étendre ce phénomène aux autres formes d'hypersensibilité immédiate. L'hypersensibilité immédiate est la forme d'allergie la plus fréquente et résulte de la synthèse d'immunoglobulines E (IgE), des anticorps spécifiques des allergènes de l'environnement. Ces immunoglobulines E se fixent sur des cellules dont les mastocytes sont les éléments clés. Le contact de l'allergène et des immunoglobulines E active les mastocytes qui libèrent les médiateurs de l'inflammation : c'est la dégranulation des mastocytes. On regroupe sous le nom d'anaphylaxie et de maladies atopiques toutes les manifestations cliniques liées aux phénomènes d'hypersensibilité immédiate, qui résultent de la production d'immunoglobulines E, les grands coupables de l'allergie. Toutefois, l'anaphylaxie correspond à un mécanisme physiopathologique indépendant de tout facteur héréditaire (c'est par exemple, le choc anaphylactique dus aux venins d'hyménoptères), tandis que l'atopie traduit une aptitude génétique particulière à produire en excès des immunoglobulines E dirigées contre diverses substances naturelles de l'environnement atmosphérique (les pollens, les moisissures, les polluants), domestique (les acariens, les blattes, les mammifères) ou professionnel, et aussi contre les aliments ; il en résulte des allergies respiratoires (asthme, rhinites), cutanées (urticaires, eczéma atopique), ophtalmiques et digestives.

Dans ces pathologies, deux points doivent être cités :

1- les évènements en cascade déclenchés par la fixation du complexe allergène - IgE sur différentes populations cellulaires et notamment sur les mastocytes

2- le rôle des cytokines qui, par l'intermédiaire de la synthèse des IgE, participent à la phase de sensibilisation, d'une part, et à la pérennisation de la réaction allergique d'autre part.

Aujourd'hui, les traitements qui endiguent les syndromes d'origine allergique font partie de l'arsenal anti-inflammatoire (anti-histaminiques et corticoïdes). La place prépondérante prise par les cytokines dans les mécanismes fondamentaux de l'hypersensibilité immédiate ouvre des perspectives pour de nouvelles thérapies ciblées : on espère notamment faire basculer l'équilibre Th1/Th2 en faveur de la voie Th1 et moduler la signalisation cellulaire induite par les cytokines lorsqu'elles se fixent sur leurs récepteurs situés sur les lymphocytes, mais aussi sur les mastocytes et sur les éosinophiles.

La présente invention est relative à un mélange vaccinal comportant 2 peptides dénommés A16E et A16G, spécifiques du parasite *Leishmania*, associés à un adjuvant. L'invention concerne également l'utilisation des dits peptides et dudit adjuvant comme réactifs de diagnostic in vitro et in vivo, et comme réactifs induisant une stimulation des lymphocytes T de type Th1 assurant une inhibition du développement des lymphocytes de type Th2 notamment pour la prévention et le traitement d'affections liées à un état d'hypersensibilité immédiate donc un état immunitaire de type Th2.

Les deux peptides A16E et A16G présentent les séquences en acides aminés suivantes :

A16E : (16 acides aminés) :

A-A-R-S-A-R-S-R-E-G-Y-S-L-T-D-E

A16G : (16 acides aminés) :

A-A-S-S-T-P-S-P-G-S-G-C-E-V-D-G

Dans le composé peptidique A16E, L peut être indifféremment remplacé par I, et S par C.
Dans le composé peptidique A16G, C peut être indifféremment remplacé par S, et S par C.

Le mélange selon l'invention peut également être inclus dans n'importe quel polypeptide.

Les peptides obtenus par synthèse sont construits en octopus ou sont rendus immunogéniques par liaisons à des porteurs (grosses molécules type KLH), et sont administrés aux mammifères en présence d'un adjuvant, de préférence le muramyl dipeptide (MDP) : ce mélange constitue le complexe peptidique vaccinal thérapeutique.

De façon préférentielle, le rapport protéine/adjuvant est compris entre 1/0,1 et 116.

Dans le cas d'une infection par des leishmanies, les études effectuées chez le chien ont permis de déterminer la dose vaccinale optimale à 100μg de muramyl dipeptide pour 50 μg de protéines injectées. On observe toutefois un début de réponse dès 30 μg de protéines injectées.

Le mécanisme d'action spécifique du complexe peptidique obtenu selon l'invention est vérifié à l'aide de méthodes classiques permettant le dosage des peptides, leur identification et à l'aide de méthodes plus spécifiques démontrant que le complexe peptidique innovant agit soit par immunostimulation du système lymphocytaire de type Th1, soit par immunomodulation d'un type Th2 vers un type Th1.

Pour chaque mammifère étudié (chien par exemple), une analyse sérologique est effectuée avec les peptides A16E et/ou A16G.

Un examen parasitologique est effectué à partir d'échantillon prélevé directement sur le candidat étudié, chien par exemple.

Un frottis à partir de la ponction de moelle osseuse est effectué sur lame. Ce frottis, une fois fixé au méthanol est coloré au May Grümwald Giemsa et observé au microscope à immersion (x 1000).

Des prélèvements de moelle osseuse sont mis en culture dans le milieu de culture biphasique NNN (Novy and Mac Neal, 1904, 4. Infec. Dis., 1 :1-30) dont du RPMI 1640 additionné de 20% de sérum de veau foetal décomplémenté constitue la phase liquide. Des repiquages à l'aveugle sont réalisés tous les quatre à six jours. Les cultures sont régulièrement observées en microscopie photonique (x 400) pendant 20 mn.

Les parasitémies sont quantifiées comme suit :

+/- : formes allongées réfringentes immobiles
+ : 1 à 5 formes promastigotes mobiles/champs
++ : > 5 formes promastigotes mobiles/champs
+++ : culture à confluence

Mise en évidence de l'implication d'une immunité à médiation cellulaire de type Th1:

Des leishmanies de formes promastigotes sont cultivées dans des milieux de culture standards. Des parasites de fin de phase exponentielle (6-7 jours) sont récoltés. Le culot parasitaire est lavé trois fois par centrifugation (2500g, 15mn, 4°C) en tampon PBS.

Après avoir vérifié la viabilité des parasites à l'aide d'un colorant vital (le bleu de trypan), une suspension contenant $2 \times 10^8$ parasites par ml est inactivée en tampon PBS contenant 0,01% de merthiolate (Pinelli et al., 1994, Infect. Immun. 62 : 229-235). Ceci constitue les leishmanines pour le test d'intradermoréaction (IDR).

L'étude de la réponse immunitaire de type Th1 ci-après est effectuée sur des chiens.

Les chiens sont placés en décubitus latéral et une tonte délicate et non irritante est effectuée sur zone thoracique d'environ 5 cm sur 10 cm en arrière du coude. Quatre cercles de 10mm de diamètre sont délimités à l'aide d'un feutre

On injecte au centre des cercles 0,1 ml de solution en intradermo-injection. Deux cercles reçoivent de la solution de leishmanines et les 2 autres cercles reçoivent de la solution saline merthiolée en contrôle négatif. La lecture de l'intrà-dermo réaction (IDR) s'effectue 48 heures plus tard au moyen d'une réglette allergologue.

Le test est considéré positif si la moyenne de deux diamètres d'induration observée est supérieure ou égale à 5 mm. L'observation d'un érythème sans induration sera considérée comme un test négatif (Pinelli et al., 1994, Infect. Immun., 62: 229-335 ; Marty et al., 1994, trans. Roy. Soc. Trop. Med. Hyg., 88, 658-659).

Les IDR peuvent être également réalisées avec une solution de peptides A16E et A16G ou de leurs dérivés et de MDP, par exemple dans des proportions de 10 µg de peptides pour 20µg de MDP.

La présente invention concerne donc également un produit de diagnostic in vivo mettant en évidence un état d'hypersensibilité retardée immunitaire de type Th1 par l'utilisation de peptides A16E et A16G et leurs dérivés en intra-dermo réaction chez le mammifère.

On peut également procéder au dosage du monoxyde d'azote (NO) pour connaître l'activité destructrice des monocytes des Leishmanies. La synthèse de NO par les monocytes est en effet un signe de destruction des leishmanies par les monocytes ayant été activés par les cytokines de type interféron gamma (IFNγ).

Le NO possède une haute réactivité chimique. En présence d'eau et d'oxygène, cette molécule est rapidement oxydée de façon stochiométrique et forme ainsi des nitrites (NO2-) selon la réaction : $4 \, NO° + O_2 + 2H_2O \, --- \, 4 \, NO_2^- + 4 \, H^+$ Les nitrites s'accumulent dans les milieux et sont facilement détectables chimiquement par la méthode de Griess.

A 50 µl de surnageant de cultures de monocytes à tester, on ajoute 60 µl de Griess B (N-(1-Naphtyl)ethyl-enediamine 0,3%). La réaction colorimétrique se développe à l'abri de la lumière pendant 2 minutes. Les densités optiques obtenues à 540 nm sont corrigées par la soustraction des DO obtenues sur les puits contenant le milieu de culture seul.

Les valeurs obtenues sont reportées sur une courbe étalon (DO = $f(NO_2^-)$) réalisée à partir de concentrations connues de $NO_2^-$.

Pour ce test, les monocytes et les lymphocytes sont isolés à partir de sang veineux des chiens. Les monocytes sont mis en culture pendant 3 jours à raison de $10^5$ cellules par puits dans les chambres de culture (Labtek) dans un milieu RPMI 1640 complet (contenant 25mM d'HEPES ; 2 mM de L-glutamine, 100 U de pénicilline par ml), à 37°C dans une atmosphère humide contenant 5% de $CO_2$. Après 3 jours de culture, les macrophages sont lavés en milieu RPMI complet, additionnés de milieu frais. Les cellules sont mises en incubation soit seules, soit en présence de 5µg de peptides, soit en présence de lymphocytes autologues.

Lorsqu'ils sont utilisés, les lymphocytes cultivés séparément sont lavés, comptés et additionnés aux macrophages dans la proportion de 2 lymphocytes par macrophage.

On effectue également un test de prolifération lymphocytaire.

Les cellules mononucléées du sang périphérique (PBMC) des chiens sont séparées sur gradient de Ficoll (densité 1,078) par centrifugation à 800g pendant 20 mn à température ambiante. Ces cellules sont mises en culture en plaque de 96 puits à la concentration de $2.10^5$ cellules par puits en présence de 2µg par ml de Concanavalin A (Sigma), de 5 µg par ml de ES P (excrétion - sécrétion promastigotes) ou 20 ml de surnageants de culture récoltés en phase stationnaire de la croissance des promastigotes (SP) par puits, et en absence de tout additif dans un volume de 200 ml de milieu RPMI 1640 contenant 5% de sérum de veau foetal décomplémenté, 2 mM de L-glutamine, 100 U de pénicilline par ml, 100 mg de streptomycine par ml. Les concentrations optimales d'antigènes et de mitogènes ont été déterminées dans des expériences préalables. Les PBMC sont incubées pendant 72 heures dans une atmosphère humide à 37°C en présence de 5% de $CO_2$ puis pendant 20 heures avec 0,5 µCi de $^3$H thymidine. Les cellules sont récoltées sur filtre et l'incorporation de la radioactivité est déterminée par comptage en liquide scintillant (compteur β). Tous les tests sont réalisés en triplicata.

Une méthode immunohistochimique plus rapide et plus sensible utilisant le BrdU (5-bromo-2'-désoxyuridine), un analogue structural de la thymidine est aussi utilisée pour mesurer la prolifération cellulaire (BrdU, cell prolifération détection kit III, Boehringer Mannheim, Allemagne). Dans nos expériences le BrdU est additionné pendant 18 heures après 72 heures d'incubation. Les cellules qui ont incorporé le BrdU dans leur ADN sont facilement détectables en présence d'un anticorps monoclonal dirigé contre le BrdU.

Les réponses prolifératives sont exprimées en indices de stimulation qui représentent le rapport de la moyenne de prolifération après stimulation sur la moyenne de prolifération en absence d'antigène.

La prolifération lymphocytaire a aussi été estimée par des lectures visuelles en microscopie photonique (- : négatif ; +/- : légère prolifération ; + : petite prolifération en moins de 5 points par champs microscopique ; ++ : prolifération moyenne en plus de 5 points ; +++ : forte prolifération).

Parallèlement à l'étude de l'activation spécifique des lymphocytes T de type Th1 par intradermoréaction, dosage de NO (le NO est synthétisé par les monocytes activés par les cytokines des cellules T de type Th1) et prolifération lymphocytaire, un suivi sérologique par immunofluorescence classique utilisant des lames coatées par des promastigotes (méthode de référence sérologique pour la leishmaniose canine) est effectué.

Pour le déroulement des études, d'autres techniques particulières ont été utiles.

Méthode d'épreuve infectieuse :

L'épreuve infectieuse consiste à injecter en intraveineuse $10^6$ promastigotes en phase métacyclique traités au complément de chien sain et de $5.10^6$ macrophages péritonéaux de chien sain, infectés in vitro par des amastigotes. Les promastigotes et les macrophages infectés sont dilués dans du sérum physiologique stéril pour un volume final de 1,5 ml. Ce mélange est effectué juste avant l'injection.

Détection des immunoglobulines d'isotype IgG2 de chiens, spécifiques des peptides A16E et A16G:

Cette détection s'effectue par méthode ELISA selon la technique de microtitration de Kweider et al (J. Immunol, 1987, 138, 299) en utilisant un conjugué anti IgG2. Pour cette méthode, les peptides sont biotinylés avant coating sur microplaques. Cette liaison à de grosses molécules de type biotine a notamment pour effet de rendre les peptides A16E ou A16G ou leurs dérivés plus antigéniques.

Les peptides peuvent également être reliés par de la glutaraldéhyde ou associés à des corps de polylysine (présentation de type OCTOPUS par exemple).

Le caractère innovant du complexe peptidique selon l'invention ne réside pas seulement dans l'induction d'une réponse cellulaire spécifique de type Th1 mais également dans la production de faibles taux d'immunoglobulines d'isotype IgG particuliers comme les IgG2 chez le chien. Ces IgG particulières sont détectables par diverses méthodes in vitro, par exemple: ELISA, DOT BLOT, WESTERN BLOT, IMMUNOCHROMATOGRAPHIE, LATEX et toute autre méthode in vitro faisant intervenir un système de conjugué ou autres systèmes de visualisation de la réaction Ag - Ac. Il peut par exemple être utilisé un système ELISA sur un support plastic, et un système WESTERN BLOT sur membranes de nitrocellulose ou autres polymères faisant intervenir un conjugué enzymatique. Des supports de latex peuvent également être utilisés. Les peptides A16E, A16G et leurs dérivés peuvent également être conjugués par exemple à des radio-isotopes, des molécules fluorescentes, des molécules luminescentes ou des particules de couleurs.

En effet, certains travaux préliminaires chez l'homme (KAWANO.P et al, Parasite Immunol, 1995, 17, 451-458) et chez le chien (NIETO C.G et al, Vet Immunol and Immunopathology, 199, 67, 117-130) démontrent que les isotypes d'IgG seraient des marqueurs de la dichotomie immunitaire Th1/Th2. Plus précisément, un chien atteint de leishmaniose avec des signes cliniques probants présente un taux élevé d'anticorps principalement d'isotype IgG1 alors qu'un chien asymptomatique présente des anticorps spécifiques d'isotype IgG2. Les chiens ayant reçu notre complexe peptidique présentent des taux faibles d'IgG2 spécifiques des peptides A16E et/ou A16G, ce qui est conforme à l'expansion préférentielle de lymphocytes T de type Th1.

La détection de la présence d'isotypes d'IgG particuliers spécifiques des peptides A16E et A16G permet notamment:

- de mettre en évidence une réponse humorale dépendant de lymphocytes Th1 et donc de mettre en évidence un état immunitaire de type Th1
- de mettre en évidence un état d'hypersensibilité retardée,
- de suivre la réponse immune chez les mammifères vaccinés ou traités,
- de suivre l'efficacité d'un traitement chimiothérapeutique et/ou immunothérapeutique.

Le mélange comprenant des peptides A16E et A16G et de l'adjuvant, selon l'invention peut être administré de diverses manières. Il est cependant administré de façon préférentielle par quatre voies :

- soit par injection sous cutanée
- soit par injection intra dermique
- soit par injection intra musculaire
- soit par voie orale

D'autres modes d'administration peuvent être employés comme la voie parentérale ou intraveineuse.

De façon générale, un vaccin se présente sous une forme injectable composée d'une fraction lyophilisée que l'on reprend par une fraction liquide ou diluant. Les doses utilisées pour la prévention et l'immunothérapie sont différentes, et sont également différentes selon le mode d'injection :

- par voie sous cutanées et intra musculaires

  - injection d'une dose (50$\mu$g de peptides et 100$\mu$g d'adjuvant) chez les chiens quels que soient la race et le sexe pour un effet préventif
  - injection de demi doses (25$\mu$g de peptides et 50$\mu$g d'adjuvant) pour l'immunothérapie des chiens leishmaniens.

- par voie intra dermo :

  - injection de demi dose chez les chiens leishmaniens pour un effet préventif
  - injection de quart de dose chez les chiens leishmaniens pour un effet thérapeutique.

Les méthodes d'injections sont reprises dans les exemples d'immunothérapie et de vaccination.

RESULTATS EN IMMUNOTHERAPIE

Selon les spécialistes comme PINELLI (PINELLI, E et al, Infect Immun, 1994, 62.229-235) les chiens leishmaniens correspondant à l'activation du système lymphocytaire de type Th2 présentent une réponse en anticorps élevée.

Cette production accrue en anticorps correspond à l'hyper protéinémie et induit l'apparition d'immun complexes entraînant une atteinte rénale (augmentation de la créatinine et de l'urée sanguines).

Nous avons donc essayé de moduler vers un état Th1 en administrant à des chiens parfaitement leishmaniens par voie intra dermo des doses des complexes peptidiques. Le suivi de l'état immunitaire ainsi que l'observation clinique sont effectuées avant et après le traitement.

Exemple 1 : chienne MANON

Une chienne de race Epagneul breton âgée de 4 ans, appartenant à Mr P présente de nombreuses lésions cutanées accompagnées d'un état de fatigue général et un aspect maigre, le tout évoquant une leishmaniose canine. Le vétérinaire, le Dr LM diagnostique une leishmaniose. Ce diagnostic est confirmé par une observation directe au microscope de leishmanies à partir d'un calque cutané et une analyse sérologique qui donne un titre en immunofluorescence leishmaniose positif à 1/3200.

L'analyse de l'état immunitaire avant toute injection permet d'affirmer que le chien est bien dans un état immunitaire de type Th2 avec un titre en anticorps élevé ainsi que des tests IDR et dosages de NO négatifs. Nous instaurons une immunothérapie qui consiste à effectuer par voie intradermo 2 injections de 25 $\mu$g de peptides (1/2 A16E, ½ A16G) et 100$\mu$g d'adjuvant muramyl dipeptide chaque injection étant espacée de 3 semaines.

Une semaine après la seconde injection la chienne MANON retrouve de l'appétit et une certaine vitalité. Le Dr LM commence à observer une légère amélioration cutanée.

Un mois après la dernière injection, MANON a retrouvé un aspect clinique normal avec notamment une augmentation de poids de 1 kg et une disparition à 80% de toutes lésions cutanées. L'analyse de l'état immunitaire permet d'affirmer une baisse de titre en anticorps anti leishmania qui descend à 1/400 en immunofluorescence. Parallèlement, l'IDR (IDR effectuée avec des leishmanines et également avec les peptides A16E et A16G), le dosage de NO et la prolifération lymphoblastique se sont positivés. De plus, après traitement, la chienne MANON présente des IgG2 spécifiques des 2 peptides, IgG2 dosées par méthode ELISA et Western Blot. Ces IgG2 spécifiques étaient absentes avant tout traitement. Une recherche de parasites par culture sur milieu NNN s'avère négative. 8 mois après le traitement la chienne MANON ne présente aucune modification. Les analyses biologiques permettent d'affirmer que MANON est toujours en état immunitaire Th1.

Exemple 2 : Chien PEPPONE

Un chien mâle de race Griffon, PEPPONE âgé de 5 ans appartenant à Mr B présente les signes cliniques spécifiques de la leishmaniose. Selon le Dr GH, présence de nombreuses squames brillantes, dépilation péri oculaire droite, lésions ulcéreuses au niveau des 2 coudes antérieurs et un état de fatigue prononcé. Les analyses biologiques avec notamment une sérologie leishmaniose positive au 1/400 en immunofluorescence confortent le diagnostic clinique. Une immunothérapie consistant à effectuer par voie intradermo 3 injections de 25μg de composé vaccinal peptidique (1/2 A16E, 1/2 A16G) et de 100μg d'adjuvant muramyl dipeptide, chaque injection étant espacée de 10 jours, est instaurée. L'analyse de l'état immunitaire avant toute injection démontre que le chien PEPPONE développe un système immunitaire de type Th2 avec une parasitémie fortement positive à partir de la moelle osseuse.

Un mois après la dernière injection, les signes cliniques leishmaniens de PEPPONE rétrocèdent avec notamment une cicatrisation des lésions ulcéreuses, une disparition importante des squames ainsi qu'une dépilation péri oculaire presque inexistante. La sérologie présente toujours un titre en immunofluorescence égal au 1/400. Par contre l'analyse de la réponse cellulaire permet d'affirmer que PEPPONE présente un état Th1 actif avec une IDR (IDR effectuée avec des leishmanines et également avec les peptides A16E et A16G), un dosage de NO et une prolifération lymphoblastique positifs.

Parallèlement, la parasitémie s'est négativée (culture de moelle osseuse en milieu NNN).

Au niveau humoral, le chien PEPPONE présente des IgG2 spécifiques des peptides A16E et A16G après le traitement, IgG2 dosées en ELISA et en Western Blot.

La présente invention consiste donc bien en un mélange thérapeutique qui induit le passage d'un état immunitaire de type Th2 avec production importante d'anticorps qui exacerbe les manifestations cliniques vers un état immunitaire de type Th1 entraînant la guérison.

Le mélange composé des deux peptides A16E et A16G et de l'adjuvant muramyl dipeptide constitue bien un complexe thérapeutique.

RESULTATS EN VACCINATION

Afin d'évaluer l'efficacité du mélange vaccinal selon l'invention, le composé vaccinal est testé sur 5 chiens parfaitement sains. Ces 5 chiens présentent une sérologie leishmaniose négative, une parasitémie négative ainsi que des tests de réponse cellulaire spécifique à *Leishmania* parfaitement négatifs. De plus, aucun des 5 chiens ne présente des IgG2 spécifiques d'un ou des 2 peptides A16E et/ou A16G.

Ces 5 chiens vivent dans un endroit exempt de tout phlébotome. Nous définissons 3 groupes de chiens :

1- groupe témoins (placebo)

- témoin négatif : chien LILI de race Pointer, sexe femelle, âge : 3 ans
- Témoin adjuvant seul : chienne MIMI de race Epagneul Breton, sexe femelle. Age : 6 ans

2- Groupe chiens vaccinés avec peptides seuls (50μg) et adjuvant muramyl dipeptide (100μg).

- chienne MAMA de race braque de Weymar, sexe femelle. Age : 2 ans et demi => peptide A16E
- chienne NUNU de race Pointer, sexe mâle. Age : 2 ans et demi => peptide A16G

3- groupe chien vacciné avec peptides A16E et A16G (25μg de chaque peptide) et adjuvant muramyl dipeptide (100ug).

- Chien LEON de race Epagneul Breton, sexe mâle. Age : 4 ans.

Le schéma d'injection vaccinale est le suivant:

| | J0 | | | J28 | | | J84 |
|---|---|---|---|---|---|---|---|
| | 1ère injection | ➔ | 4 semaines ➔ | 2ième injection ➔ | 8 semaines | ➔ | épreuve infectieuse |
| | 1 dose en | | | 1 dose en | | | |
| | sous cutanée | | | sous cutanée | | | |

Un suivi clinique des 5 chiens est effectué toutes les deux semaines. Les analyses biologiques sont schématisées de la façon suivante :

J0_____J28_____J84_____J84 +2 mois

1ère injection       2nde injection       épreuve

J14       J56       infectieuse

Analyses biologiques       analyses biologiques       analyses biologiques

Les analyses biologiques consistent en :

- analyses biochimiques : urée, créatines, transaminases
- analyses hématologiques : numération, formule
- sérologie leishmaniose : immunofluorescence quantitative anti leishmania, dosage par ELISA des IgG2 spécifiques des peptides A16E et/ou A16G
- tests de réponse cellulaire : IDR (Intra Dermo Réaction) effectuée avec des leishmanines et également avec les peptides A16E et A16G, dosage du NO et prolifération lymphoblastique

il faut ajouter à ces analyses, la recherche des *Leishmania* par observation directe au microscope et culture sur milieu NNN à partir de moelle osseuse après l'épreuve infectieuse.

RESULTATS :

Suivi clinique:

Aucune manifestation clinique importante n'est apparue pendant toute cette étude. Il faut noter un léger amaigrissement et l'apparition de quelques squames chez la chienne LILI, 4 mois après l'épreuve infectieuse.

Suivi biologique :

1- Les paramètres biochimiques et hématologiques sont restés normaux tout au long de cette étude.
2- Sérologie leishmaniose et parasitémie :
Avant toute injection, les 5 chiens présentent des sérologies et parasitémies négatives. Le tableau ci-après montre les réponses sérologiques obtenues lors de nos expériences et le suivi de la parasitémie (analyses effectuées 2 mois et 12 mois après l'épreuve infectieuse).

| | | SEROLOGIE | | PARASITEMIE (sur ponction de moelle) | |
|---|---|---|---|---|---|
| | Chiens | IF quantitative | ELISA IgG2 | Examen direct | Culture sur Milieu NNN |
| Chiens témoins | LILI | - | - | + | + |
| | MIMI | - | - | + | ++ |

(suite)

| | | SEROLOGIE | | | PARASITEMIE (sur ponction de moelle) | |
|---|---|---|---|---|---|---|
| | Chiens | IF quantitative | ELISA IgG2 | Examen direct | Culture sur Milieu NNN | |
| Chiens immunisés | MAMA (A16E) | - | + (0.700) | - | - | |
| | NUNU (A16G) | - | + (0.520) | - | - | |
| | LEON (A16E+A16G) | - | + (0.780) | - | - | |

Légende:
IF: immunofluorescence (considérée positive si le titres est ≥1/100)
ELISA : Cutt off=0.300 en DO (densité optique)
Parasitémie : culture sur milieu NNN:
    - = absence
    ++ = plus de 5 formes promastigotes mobiles/champs

Seuls les chiens immunisés présentent des anticorps spécifiques d'isotype IgG2 (ELISA envers les peptides correspondants) ainsi que des parasitémies négatives. Il faut noter une légère apparition d'anticorps totaux (1/200 en IF) chez tous les chiens après l'épreuve infectieuse.

Seuls les chiens témoins (LILI et MIMI) présentent des parasitémies positives ainsi qu'une absence d'anticorps spécifiques IgG2 anti peptides.

• Réponse à médiation cellulaire

Avant toute injection, les 5 chiens présentent une réponse à médiation cellulaire envers *Leishmania infantum* parfaitement négative. Selon le tableau suivant, seuls les chiens immunisés présentent des tests de prolifération lymphoblastique, des IDR positives associées à la production de NO par les monocytes.

Le tableau suivant montre les réponses obtenues de type cellulaire (analyses effectuées 2 mois après l'épreuve infectieuse).

| | CHIENS | Leishmanines | IDR A16E | A16G | Dosage Du NO (en μM) | Test de prolifération lymphoblastique |
|---|---|---|---|---|---|---|
| Chiens Témoins | LILI | + | - | - | 0,3 | - 1,1 (3) |
| | MIMI | - | - | - | 0,2 | - 1,2(3,1) |
| Chiens Immunisés | MAMA (A16E) | + | + | limite | 2,6 | + 2,1 (3,1) |
| | NUNU (A16G) | + | Limite | + | 2,8 | ++ 3,1 (3,6) |
| | LEON (A16E+ A16G) | + | + | + | 4,2 | +++ 3,7 (3,8) |

Légende:
• IDR : Le test Intra Dermo Réaction est considéré positif (+) si l'induration est ≥ 5 mm 48h après l'intradermoinjection.
• Dosage du NO:
• Test de prolifération lymphoblastique : les résultats sont exprimés par lecture en microscopie photonique et en indices de stimulation (entre parenthèses, indice de stimulation + Concanavalin A).

De par cette analyse, les composés peptidiques avec l'adjuvant induisent bien une immunité à médiation cellulaire de type Th1 protecteur auquel il faut ajouter une induction d'anticorps d'isotype IgG2. Le mélange des deux peptides à concentration égale donne une réponse cellulaire plus marquée (taux de NO synthétisé par les monocytes activés élevé)

Le mélange composé des deux peptides A16E et A16G et de l'adjuvant muramyl dipeptide constitue bien un complexe vaccinal.

**Revendications**

1.  Mélange vaccinal thérapeutique destiné à la prévention ou au traitement d'affections chez les mammifères et en particulier chez l'homme, les canidés, les félidés et les équidés dont l'immunité protectrice dépend de la stimulation de lymphocytes de type Th1 et notamment d'un état d'hypersensibilité retardée, **caractérisé en ce qu'**il contient :

    - un peptide de la séquence d'acides aminés suivante (A16E - SEQ ID N°1) :

    A-A-R-S-A-R-S-R-E-G-Y-S-L-T-D-E

    séquence dans laquelle L peut être remplacée par 1, et S par C
    - et un peptide de la séquence d'acides aminés suivante (A16G - SEQ ID N°79) :

    A-A-S-S-T-P-S-P-G-S-G-C-E-V-D-G

séquence dans laquelle C peut être remplacée par S, et S par C
- et un adjuvant qui induit de préférence une réponse à médiation cellulaire.

2. Mélange vaccinal thérapeutique selon la revendication 1 **caractérisé en ce que** les séquences A16E et A16G sont associées de préférence à la même concentration

3. Mélange vaccinal thérapeutique selon la revendication 1 ou 2 **caractérisé en ce que** l'adjuvant est du muramyl dipeptide

4. Mélange vaccinal thérapeutique selon la revendication 3 **caractérisé en ce que** le muramyl dipeptide est associé aux peptides A16E, ou A16G, dans un rapport en poids peptide sur adjuvant de 1/0,1 à 1/6

5. Mélange vaccinal thérapeutique selon la revendication 3 ou 4 **caractérisé en ce que** le muramyl dipeptide est associé aux peptides A16E, ou A16G, dans un rapport de 50$\mu$g de protéines pour 100$\mu$g de muramyl dipeptide

6. Mélange vaccinal thérapeutique selon l'une quelconque des revendications 1 à 5, **caractérisé en ce que** les peptides A16E ou A16G sont construits en octopus ou sont reliés à des grosses molécules de type KLH ou lipides, ou incluses dans des liposomes pour les rendre immunogènes.

7. Mélange vaccinal thérapeutique selon l'une quelconque des revendications 1 à 6, conditionné sous une forme telle qu'il peut être administré par différentes voies : cutanée, intradermique, intramusculaire, intraveineuse, parentale et orale.

8. Utilisation du mélange vaccinal selon l'une quelconque des revendications 1 à 7 pour la fabrication d'un médicament, ou d'un vaccin ou d'un réactif de diagnostic in vivo ou in vitro pour l'induction ou le diagnostic chez le mammifère d'une activation de l'immunité à médiation cellulaire dépendante de lymphocytes T de type Th1.

9. Utilisation du mélange vaccinal selon l'une quelconque des revendications 1 à 7 pour la fabrication d'un médicament, ou d'un vaccin ou d'un réactif de diagnostic in vivo ou in vitro pour l'induction ou le diagnostic chez le mammifère du passage d'un état immunitaire de type Th2 vers un état immunitaire de type Th1.

10. Utilisation du mélange vaccinal selon l'une quelconque des revendications 1 à 7 pour la fabrication d'un médicament, ou d'un vaccin ou d'un réactif de diagnostic in vivo ou in vitro pour l'induction ou le diagnostic chez le mammifère d'isotypes d'anticorps spécifiques, comme les IgG2 chez le chien, de l'immunité à médiation cellulaire dépendante de lymphocytes T de type Th1.

11. Kit de diagnostic in vitro comportant le mélange vaccinal selon l'une quelconque des revendications 1 à 7 **caractérisé en ce que** les peptides A16E et A16G sont reliés à des grosses molécules de type biotine ou polylysine pour les rendre plus antigéniques.

12. Kit de diagnostic in vitro comportant le mélange vaccinal selon l'une quelconque des revendications 1 à 7, **caractérisé en ce que** les peptides A16E et A16G sont reliés par de la glutaraldéhyde.

13. Kit de diagnostic in vitro selon la revendication 11 ou 12, **caractérisé en ce que** les peptides A16E et A16G sont liés à un support solide.

14. Kit de diagnostic in vitro selon la revendication 13 **caractérisé en ce que** les peptides A16E et A16G sont liés à des membranes de nitrocellulose ou autres polymères, des supports de latex et divers matériels de plastic (polymère).

15. Kit de diagnostic in vitro selon l'une quelconque des revendications 11 à 14, **caractérisé en ce que** les peptides A16E et A16G sont conjugués à des radio-isotopes, des molécules fluorescentes, des molécules luminescentes, des enzymes et des particules de couleur.

**Claims**

1. A therapeutic vaccine mixture intended for the prevention or treatment of diseases in mammals and in particular humans, canidae, felidae, and equidae, whose protective immunity depends on the stimulation of lymphocytes of Th1 type and in particular a state of delayed hypersensitivity, **characterised in that** it contains:

- a peptide of the following sequence of amino acids (A16E - SEQ ID No 1):

A-A-R-S-A-R-S-R-E-G-Y-S-L-T-D-E, in which sequence L can be replaced by I, and S by C

- a peptide of the following sequence of amino acids (A16G - SEQ ID No 79):

A-A-S-S-T-P-S-P-G-S-G-C-E-V-D-G, in which sequence C can be replaced by S, and S by C; and

- an adjuvant which preferably induces a cell-mediated response.

2. A therapeutic vaccine mixture according to claim 1 **characterised in that** the sequences A16E and A16G are preferably associated with the same concentration.

3. A therapeutic vaccine mixture according to claim 1 or claim 2 **characterised in that** the adjuvant is muramyl dipeptide.

4. A theurapeutic vaccine mixture according to claim 3 **characterised in that** the muramyl dipeptide is associated with the peptides A16E or A16G in a ratio by weight peptide to adjuvant of 1/0.1 to 1/6.

5. A theurapeutic vaccine mixture according to claim 3 or claim 4 **characterised in that** the muramyl dipeptide is associated with the peptides A16E or A16G in a ratio of 50 $\mu$g of proteins for 100 $\mu$g of muramyl dipeptide.

6. A theurapeutic vaccine mixture according to any one of claims 1 to 5 **characterised in that** the peptides A16E or A16G are constructed in octopus form or are joined to large molecules of KLH type or lipids or included in liposomes to make them immunogenic.

7. A theurapeutic vaccine mixture according to any one of claims 1 to 6 packaged in a form such that it can be administered in different ways: cutaneous, intradermal, intramuscular, intravenous, parenteral or oral.

8. Use of the vaccine mixture according to any one of claims 1 to 7 for the production of a medicine or a vaccine or an in vivo or in vitro diagnostic reagent for induction or diagnosis in mammals of an activation of cell-mediated immunity depending on T lymphocytes of Th1 type.

9. Use of the vaccine mixture according to any one of claims 1 to 7 for the production of a medicine or a vaccine or an in vivo or in vitro diagnostic reagent for induction or diagnosis in mammals of the passage from an immune state of Th2 type to an immune state of Th1 type.

10. Use of the vaccine mixture according to any one of claims 1 to 7 for the production of a medicine or a vaccine or an in vivo or in vitro diagnostic reagent for induction or diagnosis in mammals of isotypes of specific antibodies such as IgG2 in dogs, of cell-mediated immunity depending on T lymphocytes of Th1 type.

11. An in vitro diagnosis kit comprising the vaccine mixture according to any one of claims 1 to 7 **characterised in that** the peptides A16E and A16G are joined to large molecules of biotin or polylysine type to make them more antigenic.

12. An vitro diagnosis kit comprising the vaccine mixture according to any one of claims 1 to 7 **characterised in that** the peptides A16E and A16G are joined by glutaraldehyde.

13. An vitro diagnosis kit according to claim 11 or claim 12 **characterised in that** the peptides A16E and A16G are bonded to a solid support.

14. An vitro diagnosis kit according to claim 13 **characterised in that** the peptides A16E and A16G are bonded to membranes of nitrocellulose or other polymers, latex supports and various plastic materials (polymer).

15. An in vitro diagnosis kit according to any one of claims 11 to 14 **characterised in that** the peptides A16E and A16G are conjugated to radioisotopes, fluorescent molecules, luminescent molecules, enzymes and coloured particles.

**Patentansprüche**

1. Therapeutische Impfstoffmischung, die zur Vorbeugung oder Behandlung von Erkrankungen bei Säugetieren und insbesondere beim Menschen, bei Hunden, bei Katzen und bei Pferden bestimmt ist, deren Schutzimmunität von der Stimulation von Lymphozyten des Typs Th1 und insbesondere von einem verzögerten Überempfindlichkeitszustand abhängt, **dadurch gekennzeichnet, dass** sie enthält:

   - ein Peptid mit folgender Aminosäurensequenz (A16E - SEQ ID Nr. 1):

      A-A-R-S-A-R-S-R-E-G-Y-S-L-T-D-E

   einer Sequenz, in der L durch I und S durch C ersetzt werden kann,
   - und ein Peptid mit folgender Aminosäurensequenz (A16G - SEQ ID Nr. 79):

      A-A-S-S-T-P-S-P-G-S-G-C-E-V-D-G

   einer Sequenz, in der C durch S und S durch C ersetzt werden kann,
   - und einem Adjuvans, das vorzugsweise eine zellvermittelte Reaktion induziert.

2. Therapeutische Impfstoffmischung nach Anspruch 1, **dadurch gekennzeichnet, dass** die Sequenzen A16E und A16G vorzugsweise mit der gleichen Konzentration verbunden sind.

3. Therapeutische Impfstoffmischung nach Anspruch 1 oder 2, **dadurch gekennzeichnet, dass** das Adjuvans Muramyldipeptid ist.

4. Therapeutische Impfstoffmischung nach Anspruch 3, **dadurch gekennzeichnet, dass** das Muramyldipeptid mit den Peptiden A16E oder A16G in einem Gewichtsverhältnis des Peptids zum Adjuvans von 1/0,1 bis 1/6 verbunden ist.

5. Therapeutische Impfstoffmischung nach Anspruch 3 oder 4, **dadurch gekennzeichnet, dass** das Muramyldipeptid mit den Peptiden A16E oder A16G in einem Verhältnis von 50 $\mu$g Proteinen auf 100 $\mu$g Muramyldipeptid verbunden ist.

6. Therapeutische Impfstoffmischung nach einem beliebigen der Ansprüche 1 bis 5, **dadurch gekennzeichnet, dass** die Peptide A16E oder A16G in Oktopusform aufgebaut und mit großen Molekülen des Typs KLH oder Lipiden verbunden oder in Liposomen eingeschlossen sind, um sie immunogen zu machen.

7. Therapeutische Impfstoffmischung nach einem beliebigen der Ansprüche 1 bis 6, die so aufbereitet ist, dass sie auf unterschiedlichem Weg verabreicht werden: kutan, intrakutan, intramuskulär, intravenös, parenteral und oral.

8. Verwendung der Impfstoffmischung nach einem beliebigen der Ansprüche 1 bis 7 für die Herstellung eines Medikaments oder eines Impfstoffs oder eines In-vivo- oder In-vitro-Diagnostikreagenzmittels für die Induktion oder die Diagnose einer Aktivierung der zellvermittelten Immunität, die von T-Lymphozyten des Typs Th1 abhängt, beim Säugetier.

9. Verwendung der Impfstoffmischung nach einem beliebigen der Ansprüche 1 bis 7 für die Herstellung eines Medikaments oder eines Impfstoffs oder eines In-vivo- oder In-vitro-Diagnostikreagenzmittels für die Induktion oder die Diagnose des Übergangs des Immunzustands des Typs Th2 in einen Immunzustand des Typs Th1 beim Säugetier.

10. Verwendung der Impfstoffmischung nach einem beliebigen der Ansprüche 1 bis 7 für die Herstellung eines Medikaments oder eines Impfstoffs oder eines In-vivo- oder In-vitro-Diagnostikreagenzmittels für die Induktion oder die Diagnose von spezifischen Antikörper-Isotypen, wie beispielsweise IgG2 beim Hund, der von T-Lymphozyten des Typs Th1 abhängigen zellvermittelten Immunität beim Säugetier.

11. In-vitro-Diagnosekit, das die Impfstoffmischung nach einem beliebigen der Ansprüche 1 bis 7 enthält, **dadurch gekennzeichnet, dass** die Peptide A16E und A16G mit großen Molekülen des Typs Biotin oder Polylysin verbunden sind, um sie stärker antigen zu machen.

**12.** In-vitro-Diagnosekit, das die Impfstoffmischung nach einem beliebigen der Ansprüche 1 bis 7 enthält, **dadurch gekennzeichnet, dass** die Peptide A16E und A16G durch Glutaraldehyd verbunden sind.

**13.** In-vitro-Diagnosekit nach Anspruch 11 oder 12, **dadurch gekennzeichnet, dass** die Peptide A16E und A16G an eine feste Trägersubstanz gebunden sind.

**14.** In-vitro-Diagnosekit nach Anspruch 13, **dadurch gekennzeichnet, dass** die Peptide A16E und A16G an Nitrozellulose-Membranen oder andere Polymere, Latex-Trägersubstanzen und verschiedene Kunststoffmaterialien (Polymere) gebunden sind.

**15.** In-vitro-Diagnosekit nach einem beliebigen der Ansprüche 11 bis 14, **dadurch gekennzeichnet, dass** die Peptide A16E und A16G mit Radio-Isotopen, fluoreszierenden Molekülen, lumineszierenden Molekülen, Enzymen und Farbpartikeln konjugiert sind.

## RÉFÉRENCES CITÉES DANS LA DESCRIPTION

**Documents brevets cités dans la description**

- FR 2000
- EP 02785499 A
- FR 0203134 W

**Littérature non-brevet citée dans la description**

- **REINER S.L et al.** *Annu Rev Immunol,* 1995, vol. 13, 151-177
- **PINELLI et al.** *Infect. Immun.,* 1994, vol. 62, 229
- **GURUNATHAN S et al.** *J.Exp Med,* 06 Octobre 1997, vol. 186, 1137-1147
- **MOREAU Y.** *Médecine et Armées,* 1994, vol. 22 (1), 89-93
- **LOHMAN et al.** *Proceedings of the National Academy of Science USA,* 1990, vol. 87, 8393-8397
- **JAFFE.C et al.** *J of Immunol,* 1990, vol. 144, 699-706
- **NOVY ; MAC NEAL.** *4. Infec. Dis.,* 1904, vol. 1, 1-30
- **PINELLI et al.** *Infect. Immun.,* 1994, vol. 62, 229-235
- **PINELLI et al.** *Infect. Immun.,* 1994, vol. 62, 229-335
- **MARTY et al.** *trans. Roy. Soc. Trop. Med. Hyg.,* 1994, vol. 88, 658-659
- **KWEIDER et al.** *J. Immunol,* 1987, vol. 138, 299
- **KAWANO.P et al.** *Parasite Immunol,* 1995, vol. 17, 451-458
- **NIETO C.G et al.** *Vet Immunol and Immunopathology,* vol. 199 (67), 117-130
- **PINELLI, E et al.** *Infect Immun,* 1994, vol. 62, 229-235

SEQUENCE LISTING

<110> ORIDAN INC.

<120> Complexe peptidique vaccinal thérapeutique destiné à la prévention et au traitement d'affections chez les mammifères

<130> B118 12EUR 3

<140> EP02785499.1
<141> 2002-09-13

<150> PCT/FR0203134
<151> 2002-09-13

<160> 156

<170> PatentIn version 3.1

<210> 1
<211> 16
<212> PRT
<213> Leishmania sp.

<220>
<221> misc_feature
<223> S peut être remplacé par C, et L par I

<400> 1

```
Ala Ala Arg Ser Ala Arg Ser Arg Glu Gly Tyr Ser Leu Thr Asp Glu
1               5                   10                  15
```

<210> 2
<211> 15
<212> PRT
<213> Leishmania sp.

<220>
<221> MISC_FEATURE
<223> S peut être remplacé par c et L par I

<400> 2

```
Ala Ala Arg Ser Ala Arg Ser Arg Glu Gly Tyr Ser Leu Thr Asp
1               5                   10                  15
```

<210> 3
<211> 15
<212> PRT
<213> Leishmania sp.

<220>

<221> MISC_FEATURE
<223> S peut être remplacé par C et L par I

<400> 3

```
Ala Arg Ser Ala Arg Ser Arg Glu Gly Tyr Ser Leu Thr Asp Glu
1               5               10                      15
```

<210> 4
<211> 14
<212> PRT
<213> Leishmania sp.

<220>
<221> MISC_FEATURE
<223> S peut être remplacé par C et L par I

<400> 4

```
Ala Ala Arg Ser Ala Arg Ser Arg Glu Gly Tyr Ser Leu Thr
1               5               10
```

<210> 5
<211> 14
<212> PRT
<213> Leishmania sp.

<220>
<221> MISC_FEATURE
<223> S peut être remplacé par C et L par I

<400> 5

```
Ala Arg Ser Ala Arg Ser Arg Glu Gly Tyr Ser Leu Thr Asp
1               5               10
```

<210> 6
<211> 14
<212> PRT
<213> Leishmania sp.

<220>
<221> MISC_FEATURE
<223> S peut être remplacé par C et L par I

<400> 6

```
Arg Ser Ala Arg Ser Arg Glu Gly Tyr Ser Leu Thr Asp Glu
1               5               10
```

<210> 7
<211> 13
<212> PRT
<213> Leishmania sp.

<220>
<221> MISC_FEATURE
<223> S peut être remplacé par C et L par I

<400> 7

```
Ala Ala Arg Ser Ala Arg Ser Arg Glu Gly Tyr Ser Leu
1               5                   10
```

<210> 8
<211> 13
<212> PRT
<213> Leishmania sp.

<220>
<221> MISC_FEATURE
<223> S peut être remplacé par C et L par I

<400> 8

```
Ala Arg Ser Ala Arg Ser Arg Glu Gly Tyr Ser Leu Thr
1               5                   10
```

<210> 9
<211> 13
<212> PRT
<213> Leishmania sp.

<220>
<221> MISC_FEATURE
<223> S peut être remplacé par C et L par I

<400> 9

```
Arg Ser Ala Arg Ser Arg Glu Gly Tyr Ser Leu Thr Asp
1               5                   10
```

<210> 10
<211> 13
<212> PRT
<213> Leishmania sp.

<220>
<221> MISC_FEATURE
<223> S peut être remplacé par C et L par I

<400> 10

```
Ser Ala Arg Ser Arg Glu Gly Tyr Ser Leu Thr Asp Glu
1               5                   10
```

<210> 11
<211> 12
<212> PRT

<213> Leishmania sp.

<220>
<221> MISC_FEATURE
<223> S peut être remplacé par C

<400> 11

```
Ala Ala Arg Ser Ala Arg Ser Arg Glu Gly Tyr Ser
1                   5                   10
```

<210> 12
<211> 12
<212> PRT
<213> Leishmania sp.

<220>
<221> MISC_FEATURE
<223> S peut être remplacé par C et L par I

<400> 12

```
Ala Arg Ser Ala Arg Ser Arg Glu Gly Tyr Ser Leu
1                   5                   10
```

<210> 13
<211> 12
<212> PRT
<213> Leishmania sp.

<220>
<221> MISC_FEATURE
<223> S peut être remplacé par C et L par I

<400> 13

```
Arg Ser Ala Arg Ser Arg Glu Gly Tyr Ser Leu Thr
1                   5                   10
```

<210> 14
<211> 12
<212> PRT
<213> Leishmania sp.

<220>
<221> MISC_FEATURE
<223> S peut être remplacé par c et L par I

<400> 14

```
Ser Ala Arg Ser Arg Glu Gly Tyr Ser Leu Thr Asp
1                   5                   10
```

<210> 15
<211> 12
<212> PRT
<213> Leishmania sp.

<220>
<221> MISC_FEATURE
<223> s peut être remplacé par C et L par I

<400> 15

```
Ala Arg Ser Arg Glu Gly Tyr Ser Leu Thr Asp Glu
1               5               10
```

<210> 16
<211> 11
<212> PRT
<213> Leishmania sp.

<220>
<221> MISC_FEATURE
<223> C peut être remplacé par S

<220>
<221> MISC_FEATURE
<223> S peut être remplacé par C

<400> 16

```
Ala Ala Arg Ser Ala Arg Ser Arg Glu Gly Tyr
1               5               10
```

<210> 17
<211> 11
<212> PRT
<213> Leishmania sp.

<220>
<221> MISC_FEATURE
<223> C peut être remplacé par S

<400> 17

```
Ala Arg Ser Ala Arg Ser Arg Glu Gly Tyr Ser
1               5               10
```

<210> 18
<211> 11
<212> PRT
<213> Leishmania sp.

<220>
<221> MISC_FEATURE

<223> S peut être remplacé par C et L par I

<400> 18

```
Arg Ser Ala Arg Ser Arg Glu Gly Tyr Ser Leu
1                   5                   10
```

<210> 19
<211> 11
<212> PRT
<213> Leishmania sp.

<220>
<221> MISC_FEATURE
<223> S peut être remplacé par C et L par 1

<400> 19

```
Ser Ala Arg Ser Arg Glu Gly Tyr Ser Leu Thr
1                   5                   10
```

<210> 20
<211> 11
<212> PRT
<213> Leishmania sp.

<220>
<221> MISC_FEATURE
<223> S peut être remplacé par c et L par I

<400> 20

```
Ala Arg Ser Arg Glu Gly Tyr Ser Leu Thr Asp
1                   5                   10
```

<210> 21
<211> 11
<212> PRT
<213> Leishmania sp.

<220>
<221> MISC_FEATURE
<223> S peut être remplacé par C et L par I

<400> 21

```
Arg Ser Arg Glu Gly Tyr Ser Leu Thr Asp Glu
1                   5                   10
```

<210> 22
<211> 10
<212> PRT
<213> Leishmania sp.

<220>
<221> MISC_FEATURE
<223> S peut être remplacé par C

<400> 22

```
Ala Ala Arg Ser Ala Arg Ser Arg Glu Gly
1               5               10
```

<210> 23
<211> 10
<212> PRT
<213> Leishmania sp.

<220>
<221> MISC_FEATURE
<223> S peut être remplacé par C

<400> 23

```
Ala Arg Ser Ala Arg Ser Arg Glu Gly Tyr
1               5               10
```

<210> 24
<211> 10
<212> PRT
<213> Leishmania sp.

<220>
<221> MISC_FEATURE
<223> S peut être remplacé par C

<400> 24

```
Arg Ser Ala Arg Ser Arg Glu Gly Tyr Ser
1               5               10
```

<210> 25
<211> 10
<212> PRT
<213> Leishmania sp.

<220>
<221> MISC_FEATURE
<223> S peut être remplacé par C et L par I

<400> 25

```
Ser Ala Arg Ser Arg Glu Gly Tyr Ser Leu
1               5               10
```

<210> 26
<211> 10

<212> PRT
<213> Leishmania sp.

<220>
<221> MISC_FEATURE
<223> S peut être remplacé par C et L par I

<400> 26

```
Ala Arg Ser Arg Glu Gly Tyr Ser Leu Thr
1               5                   10
```

<210> 27
<211> 10
<212> PRT
<213> Leishmania sp.

<220>
<221> MISC_FEATURE
<223> S peut être remplacé par C et L par I

<400> 27

```
Arg Ser Arg Glu Gly Tyr Ser Leu Thr Asp
1               5                   10
```

<210> 28
<211> 10
<212> PRT
<213> Leishmania sp.

<220>
<221> MISC_FEATURE
<223> S peut être remplacé par C et L par I

<400> 28

```
Ser Arg Glu Gly Tyr Ser Leu Thr Asp Glu
1               5                   10
```

<210> 29
<211> 9
<212> PRT
<213> Leishmania sp.

<220>
<221> MISC_FEATURE
<223> S peut être remplacé par C

<400> 29

```
Ala Ala Arg Ser Ala Arg Ser Arg Glu
1               5
```

<210> 30
<211> 9
<212> PRT
<213> Leishmania sp.

<220>
<221> MISC_FEATURE
<223> S peut être remplacé par C

<400> 30

```
Ala Arg Ser Ala Arg Ser Arg Glu Gly
1               5
```

<210> 31
<211> 9
<212> PRT
<213> Leishmania sp.

<220>
<221> MISC_FEATURE
<223> s peut être remplacé par C

<400> 31

```
Arg Ser Ala Arg Ser Arg Glu Gly Tyr
1               5
```

<210> 32
<211> 9
<212> PRT
<213> Leishmania sp.

<220>
<221> MISC_FEATURE
<223> S peut être remplacé par C

<400> 32

```
Ser Ala Arg Ser Arg Glu Gly Tyr Ser
1               5
```

<210> 33
<211> 9
<212> PRT
<213> Leishmania sp.

<220>

<221> MISC_FEATURE
<223> S peut être remplacé par C et L par I

<400> 33

Ala Arg Ser Arg Glu Gly Tyr Ser Leu
1               5

<210> 34
<211> 9
<212> PRT
<213> Leishmania sp.

<220>
<221> MISC_FEATURE
<223> S peut être remplacé par C et L par I

<400> 34

Arg Ser Arg Glu Gly Tyr Ser Leu Thr
1               5

<210> 35
<211> 9
<212> PRT
<213> Leishmania sp.

<220>
<221> MISC_FEATURE
<223> S peut être remplacé par C et L par I

<400> 35

Ser Arg Glu Gly Tyr Ser Leu Thr Asp
1               5

<210> 36
<211> 9
<212> PRT
<213> Leishmania sp.

<220>
<221> MISC_FEATURE
<223> S peut être remplacé par c, et L par I

<400> 36

Arg Glu Gly Tyr Ser Leu Thr Asp Glu
1               5

<210> 37
<211> 8
<212> PRT

<213> Leishmania sp.

<220>
<221> MISC_FEATURE
<223> S peut être remplacé par C

<400> 37

```
        Ala Ala Arg Ser Ala Arg Ser Arg
        1                   5
```

<210> 38
<211> 8
<212> PRT
<213> Leishmania sp.

<220>
<221> MISC_FEATURE
<223> S peut être remplacé par C

<400> 38

```
        Ala Arg Ser Ala Arg Ser Arg Glu
        1                   5
```

<210> 39
<211> 8
<212> PRT
<213> Leishmania sp.

<220>
<221> MISC_FEATURE
<223> S peut être remplacé par C

<400> 39

```
        Arg Ser Ala Arg Ser Arg Glu Gly
        1                   5
```

<210> 40
<211> 8
<212> PRT
<213> Leishmania sp.

<220>
<221> MISC_FEATURE
<223> S peut être remplacé par C

<400> 40

```
        Ser Ala Arg Ser Arg Glu Gly Tyr
        1                   5
```

<210> 41
<211> 8
<212> PRT
<213> Leishmania sp.

<220>
<221> MISC_FEATURE
<223> S peut être remplacé par C <400> 41

```
Ala Arg Ser Arg Glu Gly Tyr Ser
1               5
```

<210> 42
<211> 8
<212> PRT
<213> Leishmania sp.

<220>
<221> MISC_FEATURE
<223> S peut être remplacé par C et L par I

<400> 42

```
Arg Ser Arg Glu Gly Tyr Ser Leu
1               5
```

<210> 43
<211> 8
<212> PRT
<213> Leishmania sp.

<220>
<221> MISC_FEATURE
<223> S peut être remplacé par C et L par I

<400> 43

```
Ser Arg Glu Gly Tyr Ser Leu Thr
1               5
```

<210> 44
<211> 8
<212> PRT
<213> Leishmania sp.

<220>
<221> MISC_FEATURE
<223> S peut être remplacé C, et Lpar I

<400> 44

Arg Glu Gly Tyr Ser Leu Thr Asp
1                   5

<210> 45
<211> 8
<212> PRT
<213> Leishmania sp.

<220>
<221> MISC_FEATURE
<223> S peut être remplacé par C, et L par I

<400> 45

Glu Gly Tyr Ser Leu Thr Asp Glu
1                   5

<210> 46
<211> 7
<212> PRT
<213> Leishmania sp.

<220>
<221> MISC_FEATURE
<223> S peut être remplacé par C

<400> 46

Ala Ala Arg Ser Ala Arg Ser
1                   5

<210> 47
<211> 7
<212> PRT
<213> Leishmania sp.

<220>
<221> MISC_FEATURE
<223> S peut être remplacé par C

<400> 47

Ala Arg Ser Ala Arg Ser Arg
1                   5

<210> 48
<211> 7
<212> PRT
<213> Leishmania sp.

<220>

<221> MISC_FEATURE
<223> S peut être remplacé par C

<400> 48

```
Arg Ser Ala Arg Ser Arg Glu
1               5
```

<210> 49
<211> 7
<212> PRT
<213> Leishmania sp.

<220>
<221> MISC_FEATURE
<223> S peut être remplacé par C

<400> 49

```
Ser Ala Arg Ser Arg Glu Gly
1               5
```

<210> 50
<211> 7
<212> PRT
<213> Leishmania sp.

<220>
<221> MISC_FEATURE
<223> S peut être remplacé par C

<400> 50

```
Ala Arg Ser Arg Glu Gly Tyr
1               5
```

<210> 51
<211> 7
<212> PRT
<213> Leishmania sp.

<220>
<221> MISC_FEATURE
<223> S peut être remplacé par C

<400> 51

```
Arg Ser Arg Glu Gly Tyr Ser
1               5
```

<210> 52
<211> 7
<212> PRT
<213> Leishmania sp.

<220>
<221> MISC_FEATURE
<223> S peut être remplacé par C, et L par I

<400> 52

```
Ser Arg Glu Gly Tyr Ser Leu
1               5
```

<210> 53
<211> 7
<212> PRT
<213> Leishmania sp.

<220>
<221> MISC_FEATURE
<223> S peut être remplacé par C, et L par I

<400> 53

```
Arg Glu Gly Tyr Ser Leu Thr
1               5
```

<210> 54
<211> 7
<212> PRT
<213> Leishmania sp.

<220>
<221> MISC_FEATURE
<223> S peut être remplacé par C, et L par I

<400> 54

```
Glu Gly Tyr Ser Leu Thr Asp
1               5
```

<210> 55
<211> 7
<212> PRT
<213> Leishmania sp.

<220>
<221> MISC_FEATURE
<223> S peut être remplacé par C et L par I

<400> 55

```
Gly Tyr Ser Leu Thr Asp Glu
1               5
```

<210> 56
<211> 6
<212> PRT
<213> Leishmania sp.

<220>
<221> MISC_FEATURE
<223> S peut être remplacé par C

<400> 56

```
Ala Ala Arg Ser Ala Arg
1               5
```

<210> 57
<211> 6
<212> PRT
<213> Leishmania sp.

<220>
<221> MISC_FEATURE
<223> S peut être remplacé par C

<400> 57

```
Ala Arg Ser Ala Arg Ser
1               5
```

<210> 58
<211> 6
<212> PRT
<213> Leishmania sp.

<220>
<221> MISC_FEATURE
<223> S peut être remplacé par c

<400> 58

```
Arg Ser Ala Arg Ser Arg
1               5
```

<210> 59
<211> 6
<212> PRT
<213> Leishmania sp.

<220>

<221> MISC_FEATURE
<223> S peut être remplacé par C

<400> 59

Ser Ala Arg Ser Arg Glu
1             5

<210> 60
<211> 6
<212> PRT
<213> Leishmania sp.

<220>
<221> MISC_FEATURE
<223> s peut être remplacé par c

<400> 60

Ala Arg Ser Arg Glu Gly
1             5

<210> 61
<211> 6
<212> PRT
<213> Leishmania sp.

<220>
<221> MISC_FEATURE
<223> S peut être remplacé par C

<400> 61

Arg Ser Arg Glu Gly Tyr
1             5

<210> 62
<211> 6
<212> PRT
<213> Leishmania sp.

<220>
<221> MISC_FEATURE
<223> S peut être remplacé par C

<400> 62

Ser Arg Glu Gly Tyr Ser
1             5

<210> 63
<211> 6
<212> PRT
<213> Leishmania sp.

<220>
<221> MISC_FEATURE
<223> S peut être remplacé par C et L par I

<400> 63

```
Arg Glu Gly Tyr Ser Leu
1               5
```

<210> 64
<211> 6
<212> PRT
<213> Leishmania sp.

<220>
<221> MISC_FEATURE
<223> S peut être remplacé par C et L par I

<400> 64

```
Glu Gly Tyr Ser Leu Thr
1               5
```

<210> 65
<211> 6
<212> PRT
<213> Leishmania sp.

<220>
<221> MISC_FEATURE
<223> S peut être remplacé par C et L par I

<400> 65

```
Gly Tyr Ser Leu Thr Asp
1               5
```

<210> 66
<211> 6
<212> PRT
<213> Leishmania sp.

<220>
<221> MISC_FEATURE
<223> s peut être remplacé par C et L par I

<400> 66

```
Tyr Ser Leu Thr Asp Glu
1               5
```

<210> 67
<211> 5
<212> PRT
<213> Leishmania sp.

<220>
<221> MISC_FEATURE
<223> S peut être remplacé par C

<400> 67

```
Ala Ala Arg Ser Ala
1               5
```

<210> 68
<211> 5
<212> PRT
<213> Leishmania sp.

<220>
<221> MISC_FEATURE
<223> S peut être remplacé par C

<400> 68

```
Ala Arg Ser Ala Arg
1               5
```

<210> 69
<211> 5
<212> PRT
<213> Leishmania sp.

<220>
<221> MISC_FEATURE
<223> S peut être remplacé par C

<400> 69

```
Arg Ser Ala Arg Ser
1               5
```

<210> 70
<211> 5
<212> PRT
<213> Leishmania sp.

<220>
<221> MISC_FEATURE

<223> S peut être remplacé par R

<400> 70

```
Ser Ala Arg Ser Arg
1                 5
```

<210> 71
<211> 5
<212> PRT
<213> Leishmania sp.

<220>
<221> MISC_FEATURE
<223> S peut être remplacé par C

<400> 71

```
Ala Arg Ser Arg Glu
1                 5
```

<210> 72
<211> 5
<212> PRT
<213> Leishmania sp.

<220>
<221> MISC_FEATURE
<223> s peut être remplacé par C

<400> 72

```
Arg Ser Arg Glu Gly
1                 5
```

<210> 73
<211> 5
<212> PRT
<213> Leishmania sp.

<220>
<221> MISC_FEATURE
<223> S peut être remplacé par C

<400> 73

```
Ser Arg Glu Gly Tyr
1                 5
```

<210> 74
<211> 5

<212> PRT
<213> Leishmania sp.

<220>
<221> MISC_FEATURE
<223> S peut être remplacé par C

<400> 74

```
Arg Glu Gly Tyr Ser
1               5
```

<210> 75
<211> 5
<212> PRT
<213> Leishmania sp.

<220>
<221> MISC_FEATURE
<223> s peut être remplacé par c et L par l

<400> 75

```
Glu Gly Tyr Ser Leu
1               5
```

<210> 76
<211> 5
<212> PRT
<213> Leishmania sp.

<220>
<221> MISC_FEATURE
<223> S peut être remplacé par c et L par l

<400> 76

```
Gly Tyr Ser Leu Thr
1               5
```

<210> 77
<211> 5
<212> PRT
<213> Leishmania sp.

<220>
<221> MISC_FEATURE
<223> S peut être remplacé par C et L par l

<400> 77

Tyr Ser Leu Thr Asp
1           5

<210> 78
<211> 5
<212> PRT
<213> Leishmania sp.

<220>
<221> MISC_FEATURE
<223> S peut être remplacé par C et L par I

<400> 78


Ser Leu Thr Asp Glu
1           5

<210> 79
<211> 16
<212> PRT
<213> Leishmania sp.

<220>
<221> misc_feature
<223> C peut être remplacé par s, et S par C

<400> 79


Ala Ala Ser Ser Thr Pro Ser Pro Gly Ser Gly Cys Glu Val Asp Gly
1           5               10                  15

<210> 80
<211> 15
<212> PRT
<213> Leishmania sp.

<220>
<221> MISC_FEATURE
<223> C peut être remplacé par S et S par C

<400> 80


Ala Ala Ser Ser Thr Pro Ser Pro Gly Ser Gly Cys Glu Val Asp
1           5               10                  15

<210> 81
<211> 15
<212> PRT
<213> Leishmania sp.

<220>
<221> MISC_FEATURE
<223> C peut être remplacé par s et S par C

<400> 81

```
Ala Ser Ser Thr Pro Ser Pro Gly Ser Gly Cys Glu Val Asp Gly
1               5               10                      15
```

<210> 82
<211> 14
<212> PRT
<213> Leishmania sp.

<220>
<221> MISC_FEATURE
<223> C peut être remplacé par S et S par C

<400> 82

```
Ala Ala Ser Ser Thr Pro Ser Pro Gly Ser Gly Cys Glu Val
1               5               10
```

<210> 83
<211> 14
<212> PRT
<213> Leishmania sp.

<220>
<221> MISC_FEATURE
<223> C peut être remplacé par S et S par C

<400> 83

```
Ala Ser Ser Thr Pro Ser Pro Gly Ser Gly Cys Glu Val Asp
1               5               10
```

<210> 84
<211> 14
<212> PRT
<213> Leishmania sp.

<220>
<221> MISC_FEATURE
<223> C peut être remplacé par S et S par C

<400> 84

```
Ser Ser Thr Pro Ser Pro Gly Ser Gly Cys Glu Val Asp Gly
1               5                   10
```

<210> 85
<211> 13
<212> PRT
<213> Leishmania sp.

<220>
<221> MISC_FEATURE
<223> C peut être remplacé par S et S par C

<400> 85

```
Ala Ala Ser Ser Thr Pro Ser Pro Gly Ser Gly Cys Glu
1               5                   10
```

<210> 86
<211> 13
<212> PRT
<213> Leishmania sp.

<220>
<221> MISC_FEATURE
<223> C peut être remplacé par S et S par C

<400> 86

```
Ala Ser Ser Thr Pro Ser Pro Gly Ser Gly Cys Glu Val
1               5                   10
```

<210> 87
<211> 13
<212> PRT
<213> Leishmania sp.

<220>
<221> MISC_FEATURE
<223> C peut être remplacé par S et S par C

<400> 87

```
Ser Ser Thr Pro Ser Pro Gly Ser Gly Cys Glu Val Asp
1               5                   10
```

<210> 88
<211> 13
<212> PRT
<213> Leishmania sp.

<220>
<221> MISC_FEATURE
<223> C peut être remplacé par S et S par C

<400> 88

```
Ser Thr Pro Ser Pro Gly Ser Gly Cys Glu Val Asp Gly
1               5                   10
```

<210> 89
<211> 12
<212> PRT
<213> Leishmania sp.

<220>
<221> MISC_FEATURE
<223> C peut être remplacé par S et S par C

<400> 89

```
Ala Ala Ser Ser Thr Pro Ser Pro Gly Ser Gly Cys
1               5                   10
```

<210> 90
<211> 12
<212> PRT
<213> Leishmania sp.

<220>
<221> MISC_FEATURE
<223> C peut être remplacé par S et S par C

<400> 90

```
Ala Ser Ser Thr Pro Ser Pro Gly Ser Gly Cys Glu
1               5                   10
```

<210> 91
<211> 12
<212> PRT
<213> Leishmania sp.

<220>
<221> MISC_FEATURE
<223> C peut être remplacé par S et S par C

<400> 91

```
Ser Ser Thr Pro Ser Pro Gly Ser Gly Cys Glu Val
1               5                   10
```

<210> 92
<211> 12

<212> PRT

<213> Leishmania sp.


<220>

<221> MISC_FEATURE

<223> C peut être remplacé par S et S par C


<400> 92


```
        Ser Thr Pro Ser Pro Gly Ser Gly Cys Glu Val Asp
        1               5               10
```


<210> 93

<211> 12

<212> PRT

<213> Leishmania sp.


<220>

<221> MISC_FEATURE

<223> C peut être remplacé par S et S par C


<400> 93


```
        Thr Pro Ser Pro Gly Ser Gly Cys Glu Val Asp Gly
        1               5               10
```


<210> 94

<211> 11

<212> PRT

<213> Leishmania sp.


<220>

<221> MISC_FEATURE

<223> S peut être remplacé par c


<400> 94


```
        Ala Ala Ser Ser Thr Pro Ser Pro Gly Ser Gly
        1               5               10
```


<210> 95

<211> 11

<212> PRT

<213> Leishmania sp.


<220>

<221> MISC_FEATURE

<223> C peut être remplacé par S et S par C


<400> 95

```
Ala Ser Ser Thr Pro Ser Pro Gly Ser Gly Cys
1               5                   10
```

<210> 96
<211> 11
<212> PRT
<213> Leishmania sp.

<220>
<221> MISC_FEATURE
<223> C peut être remplacé par S et S par C

<400> 96

```
Ser Ser Thr Pro Ser Pro Gly Ser Gly Cys Glu
1               5                   10
```

<210> 97
<211> 11
<212> PRT
<213> Leishmania sp.

<220>
<221> MISC_FEATURE
<223> C peut être remplacé par S et S par C

<400> 97

```
Ser Thr Pro Ser Pro Gly Ser Gly Cys Glu Val
1               5                   10
```

<210> 98
<211> 11
<212> PRT
<213> Leishmania sp.

<220>
<221> MISC_FEATURE
<223> C peut être remplacé par S et S par C

<400> 98

```
Thr Pro Ser Pro Gly Ser Gly Cys Glu Val Asp
1               5                   10
```

<210> 99
<211> 11
<212> PRT
<213> Leishmania sp.

<220>
<221> MISC_FEATURE
<223> C peut être remplacé par S et S par C

<400> 99

```
Pro Ser Pro Gly Ser Gly Cys Glu Val Asp Gly
1               5               10
```

<210> 100
<211> 10
<212> PRT
<213> Leishmania sp.

<220>
<221> MISC_FEATURE
<223> S peut être remplacé par C

<400> 100

```
Ala Ala Ser Ser Thr Pro Ser Pro Gly Ser
1               5               10
```

<210> 101
<211> 10
<212> PRT
<213> Leishmania sp.

<220>
<221> MISC_FEATURE
<223> S peut être remplacé par C

<400> 101

```
Ala Ser Ser Thr Pro Ser Pro Gly Ser Gly
1               5               10
```

<210> 102
<211> 10
<212> PRT
<213> Leishmania sp.

<220>
<221> MISC_FEATURE
<223> C peut être remplacé par S et S par C

<400> 102

```
Ser Ser Thr Pro Ser Pro Gly Ser Gly Cys
1               5               10
```

<210> 103

<211> 10
<212> PRT
<213> Leishmania sp.

<220>
<221> MISC_FEATURE
<223> C peut être remplacé par S et S par C

<400> 103

```
Ser Thr Pro Ser Pro Gly Ser Gly Cys Glu
1               5                   10
```

<210> 104
<211> 10
<212> PRT
<213> Leishmania sp.

<220>
<221> MISC_FEATURE
<223> C peut être remplacé par S et S par C

<400> 104

```
Thr Pro Ser Pro Gly Ser Gly Cys Glu Val
1               5                   10
```

<210> 105
<211> 10
<212> PRT
<213> Leishmania sp.

<220>
<221> MISC_FEATURE
<223> C peut être remplacé par S et S par C

<400> 105

```
Pro Ser Pro Gly Ser Gly Cys Glu Val Asp
1               5                   10
```

<210> 106
<211> 10
<212> PRT
<213> Leishmania sp.

<220>
<221> MISC_FEATURE
<223> C peut être remplacé par S et S par c

<400> 106

```
Ser Pro Gly Ser Gly Cys Glu Val Asp Gly
1               5                   10
```

<210> 107
<211> 9
<212> PRT
<213> Leishmania sp.

<220>
<221> MISC_FEATURE
<223> S peut être remplacé par C

<400> 107

```
Ala Ala Ser Ser Thr Pro Ser Pro Gly
1               5
```

<210> 108
<211> 9
<212> PRT
<213> Leishmania sp.

<220>
<221> MISC_FEATURE
<223> S peut être remplacé par C

<400> 108

```
Ala Ser Ser Thr Pro Ser Pro Gly Ser
1               5
```

<210> 109
<211> 9
<212> PRT
<213> Leishmania sp.

<220>
<221> MISC_FEATURE
<223> s peut être remplacé par c

<400> 109

```
Ser Ser Thr Pro Ser Pro Gly Ser Gly
1               5
```

<210> 110
<211> 9
<212> PRT
<213> Leishmania sp.

<220>

<221> MISC_FEATURE
<223> C peut être remplacé par S et S par C

<400> 110

Ser Thr Pro Ser Pro Gly Ser Gly Cys
1                   5

<210> 111
<211> 9
<212> PRT
<213> Leishmania sp.

<220>
<221> MISC_FEATURE
<223> C peut être remplacé par S et S par C

<400> 111

Thr Pro Ser Pro Gly Ser Gly Cys Glu
1                   5

<210> 112
<211> 9
<212> PRT
<213> Leishmania sp.

<220>
<221> MISC_FEATURE
<223> c peut être remplacé par s et s par c

<400> 112

Pro Ser Pro Gly Ser Gly Cys Glu Val
1                   5

<210> 113
<211> 9
<212> PRT
<213> Leishmania sp.

<220>
<221> MISC_FEATURE
<223> C peut être remplacé par S et S par c

<400> 113

Ser Pro Gly Ser Gly Cys Glu Val Asp
1                   5

<210> 114

<211> 9
<212> PRT
<213> Leishmania sp.

<220>
<221> MISC_FEATURE
<223> C peut être remplacé par S et S par C

<400> 114

```
          Pro Gly Ser Gly Cys Glu Val Asp Gly
          1               5
```

<210> 115
<211> 8
<212> PRT
<213> Leishmania sp.

<220>
<221> MISC_FEATURE
<223> S peut être remplacé par C

<400> 115

```
          Ala Ala Ser Ser Thr Pro Ser Pro
          1               5
```

<210> 116
<211> 8
<212> PRT
<213> Leishmania sp.

<220>
<221> MISC_FEATURE
<223> S peut être remplacé par C

<400> 116

```
          Ala Ser Ser Thr Pro Ser Pro Gly
          1               5
```

<210> 117
<211> 8
<212> PRT
<213> Leishmania sp.

<220>
<221> MISC_FEATURE
<223> s peut être remplacé par C

<400> 117

```
Ser Ser Thr Pro Ser Pro Gly Ser
1                   5
```

<210> 118
<211> 8
<212> PRT
<213> Leishmania sp.

<220>
<221> MISC_FEATURE
<223> S peut être remplacé par C

<400> 118

```
Ser Thr Pro Ser Pro Gly Ser Gly
1                   5
```

<210> 119
<211> 8
<212> PRT
<213> Leishmania sp.

<220>
<221> MISC_FEATURE
<223> C peut être remplacé par S et S par C

<400> 119

```
Thr Pro Ser Pro Gly Ser Gly Cys
1                   5
```

<210> 120
<211> 8
<212> PRT
<213> Leishmania sp.

<220>
<221> MISC_FEATURE
<223> C peut être remplacé par S et S par C

<400> 120

```
Pro Ser Pro Gly Ser Gly Cys Glu
1                   5
```

<210> 121
<211> 8
<212> PRT
<213> Leishmania sp.

<220>
<221> MISC_FEATURE
<223> C peut être remplacé par S et S par C

<400> 121

```
Ser Pro Gly Ser Gly Cys Glu Val
1               5
```

<210> 122
<211> 8
<212> PRT
<213> Leishmania sp.

<220>
<221> MISC_FEATURE
<223> C peut être remplacé par s et S par C

<400> 122

```
Pro Gly Ser Gly Cys Glu Val Asp
1               5
```

<210> 123
<211> 8
<212> PRT
<213> Leishmania sp.

<220>
<221> MISC_FEATURE
<223> C peut être remplacé par S et S par C

<400> 123

```
Gly Ser Gly Cys Glu Val Asp Gly
1               5
```

<210> 124
<211> 7
<212> PRT
<213> Leishmania sp.

<220>
<221> MISC_FEATURE
<223> S peut être remplacé par C

<400> 124

```
Ala Ala Ser Ser Thr Pro Ser
1               5
```

<210> 125
<211> 7
<212> PRT
<213> Leishmania sp.

<220>
<221> MISC_FEATURE
<223> S peut être remplacé par C

<400> 125

```
Ala Ser Ser Thr Pro Ser Pro
1               5
```

<210> 126
<211> 7
<212> PRT
<213> Leishmania sp.

<220>
<221> MISC_FEATURE
<223> S peut être remplacé par C

<400> 126

```
Ser Ser Thr Pro Ser Pro Gly
1               5
```

<210> 127
<211> 7
<212> PRT
<213> Leishmania sp.

<220>
<221> MISC_FEATURE
<223> S peut être remplacé par C

<400> 127

```
Ser Thr Pro Ser Pro Gly Ser
1               5
```

<210> 128
<211> 7
<212> PRT
<213> Leishmania sp.

<220>
<221> MISC_FEATURE
<223> S peut être remplacé par C

<400> 128

Thr Pro Ser Pro Gly Ser Gly
1               5

<210> 129
<211> 7
<212> PRT
<213> Leishmania sp.

<220>
<221> MISC_FEATURE
<223> S peut être remplacé par C et C par S

<400> 129

Pro Ser Pro Gly Ser Gly Cys
1               5

<210> 130
<211> 7
<212> PRT
<213> Leishmania sp.

<220>
<221> MISC_FEATURE
<223> S peut être remplacé par C et C par S

<400> 130

Ser Pro Gly Ser Gly Cys Glu
1               5

<210> 131
<211> 7
<212> PRT
<213> Leishmania sp.

<220>
<221> MISC_FEATURE
<223> S peut être remplacé par C et C par S

<400> 131

Pro Gly Ser Gly Cys Glu Val
1               5

<210> 132
<211> 7
<212> PRT
<213> Leishmania sp.

<220>

<221> MISC_FEATURE
<223> S peut être remplacé par C et C par S

<400> 132

Gly Ser Gly Cys Glu Val Asp
1               5

<210> 133
<211> 7
<212> PRT
<213> Leishmania sp.

<220>
<221> MISC_FEATURE
<223> S peut être remplacé par C et C par S

<400> 133

Ser Gly Cys Glu Val Asp Gly
1               5

<210> 134
<211> 6
<212> PRT
<213> Leishmania sp.

<220>
<221> MISC_FEATURE
<223> S peut être remplacé par C

<400> 134

Ala Ala Ser Ser Thr Pro
1               5

<210> 135
<211> 6
<212> PRT
<213> Leishmania sp.

<220>
<221> MISC_FEATURE
<223> S peut être remplacé par C

<400> 135

Ala Ser Ser Thr Pro Ser
1               5

<210> 136
<211> 6

<212> PRT
<213> Leishmania sp.

<220>
<221> MISC_FEATURE
<223> S peut être remplacé par C

<400> 136

```
Ser Ser Thr Pro Ser Pro
1               5
```

<210> 137
<211> 6
<212> PRT
<213> Leishmania sp.

<220>
<221> MISC_FEATURE
<223> S peut être remplacé par C

<400> 137

```
Ser Thr Pro Ser Pro Gly
1               5
```

<210> 138
<211> 6
<212> PRT
<213> Leishmania sp.

<220>
<221> MISC_FEATURE
<223> S peut être remplacé par C

<400> 138

```
Thr Pro Ser Pro Gly Ser
1               5
```

<210> 139
<211> 6
<212> PRT
<213> Leishmania sp.

<220>
<221> MISC_FEATURE
<223> S peut être remplacé par C

<400> 139

```
Pro Ser Pro Gly Ser Gly
1                   5
```

<210> 140
<211> 6
<212> PRT
<213> Leishmania sp.

<220>
<221> MISC_FEATURE
<223> S peut être remplacé par C et C par S

<400> 140

```
Ser Pro Gly Ser Gly Cys
1                   5
```

<210> 141
<211> 6
<212> PRT
<213> Leishmania sp.

<220>
<221> MISC_FEATURE
<223> S peut être remplacé par C et C par S

<400> 141

```
Pro Gly Ser Gly Cys Glu
1                   5
```

<210> 142
<211> 6
<212> PRT
<213> Leishmania sp.

<220>
<221> MISC_FEATURE
<223> S peut être remplacé par C et C par S

<400> 142

```
Gly Ser Gly Cys Glu Val
1                   5
```

<210> 143
<211> 6
<212> PRT
<213> Leishmania sp.

<220>

<221> MISC_FEATURE
<223> S peut être remplacé par C et C par S

<400> 143

```
             Ser Gly Cys Glu Val Asp
             1               5
```

<210> 144
<211> 6
<212> PRT
<213> Leishmania sp.

<220>
<221> MISC_FEATURE
<223> C peut être remplacé par S

<400> 144

```
             Gly Cys Glu Val Asp Gly
             1               5
```

<210> 145
<211> 5
<212> PRT
<213> Leishmania sp.

<220>
<221> MISC_FEATURE
<223> S peut être remplacé par C

<400> 145

```
             Ala Ala Ser Ser Thr
             1               5
```

<210> 146
<211> 5
<212> PRT
<213> Leishmania sp.

<220>
<221> MISC_FEATURE
<223> S peut être remplacé par C

<400> 146

```
             Ala Ser Ser Thr Pro
             1               5
```

<210> 147
<211> 5

<212> PRT
<213> Leishmania sp.

<220>
<221> MISC_FEATURE
<223> S peut être remplacé par C

<400> 147

```
Ser Ser Thr Pro Ser
1               5
```

<210> 148
<211> 5
<212> PRT
<213> Leishmania sp.

<220>
<221> MISC_FEATURE
<223> S peut être remplacé par C

<400> 148

```
Ser Thr Pro Ser Pro
1               5
```

<210> 149
<211> 5
<212> PRT
<213> Leishmania sp.

<220>
<221> MISC_FEATURE
<223> S peut être remplacé par C

<400> 149

```
Thr Pro Ser Pro Gly
1               5
```

<210> 150
<211> 5
<212> PRT
<213> Leishmania sp.

<220>
<221> MISC_FEATURE
<223> S peut être remplacé par C

<400> 150

```
Pro Ser Pro Gly Ser
1               5
```

<210> 151
<211> 5
<212> PRT
<213> Leishmania sp.

<220>
<221> MISC_FEATURE
<223> S peut être remplacé par C

<400> 151

```
Ser Pro Gly Ser Gly
1               5
```

<210> 152
<211> 5
<212> PRT
<213> Leishmania sp.

<220>
<221> MISC_FEATURE
<223> S peut être remplacé par C, et C par S

<400> 152

```
Pro Gly Ser Gly Cys
1               5
```

<210> 153
<211> 5
<212> PRT
<213> Leishmania sp.

<220>
<221> MISC_FEATURE
<223> S peut être remplacé par C, et C par s

<400> 153

```
Gly Ser Gly Cys Glu
1               5
```

<210> 154
<211> 5
<212> PRT
<213> Leishmania sp.

<220>
<221> MISC_FEATURE
<223> S peut être remplacé par C, et C par S

<400> 154

```
Ser Gly Cys Glu Val
1               5
```

<210> 155
<211> 5
<212> PRT
<213> Leishmania sp.

<220>
<221> MISC_FEATURE
<223> C peut être remplacé par s

<400> 155

```
Gly Cys Glu Val Asp
1               5
```

<210> 156
<211> 5
<212> PRT
<213> Leishmania sp.

<220>
<221> MISC_FEATURE
<223> C peut être remplacé par s

<400> 156

```
Cys Glu Val Asp Gly
1               5
```